(19) Europäisches Patentamt
European Patent Office
Office européen des brevets

(11) Veröffentlichungsnummer : **0 463 592 B1**

(12) # EUROPÄISCHE PATENTSCHRIFT

(45) Veröffentlichungstag der Patentschrift : **17.08.94 Patentblatt 94/33**

(51) Int. Cl.$^5$ : **C07D 213/89**

(21) Anmeldenummer : **91110343.0**

(22) Anmeldetag : **22.06.91**

(54) **y,4- und 2,5-substituierte Pyridin-N-oxide, Verfahren zu deren Herstellung sowie deren Verwendung.**

(30) Priorität : **28.06.90 DE 4020570**

(43) Veröffentlichungstag der Anmeldung : **02.01.92 Patentblatt 92/01**

(45) Bekanntmachung des Hinweises auf die Patenterteilung : **17.08.94 Patentblatt 94/33**

(84) Benannte Vertragsstaaten : **AT BE CH DE DK ES FR GB GR IT LI LU NL SE**

(56) Entgegenhaltungen :
**EP-A- 0 278 452**
**EP-A- 0 278 453**
**CHEMICAL ABSTRACT, vol. 67, no. 21, 20 November 1967, no.98869w, Columbus, Ohio, US; & MILOS CHVAPIL et al. : "Mechanism of the antifibrous effect of poly(vinyl-pyridine-N-oxide)" & Prac.Lek. 19(5), 206-11 (1967)**

(56) Entgegenhaltungen :
**JOURNAL OF PHYSICAL ORGANIC CHEMISTRY vol. 3, 489-92 (1990); B. BRYCKI et al.: "Kinetics and mechanism of acid hydrolysis of 2-carboethoxypyridine N-oxides"**
**European Journal of Biochemistry, vol. 138, no. 2, pages 239-45**
**CHEMICAL ABSTRACTS vol. 97, no. 25, 20. Dezember 1982, no. 215892f, Columbus, Ohio, US; & JP 82109792 (BANYU PHARMACEITICAL CO. LTD.) 08.07.1982**

(73) Patentinhaber : **HOECHST AKTIENGESELLSCHAFT**
**D-65926 Frankfurt (DE)**

(72) Erfinder : **Baader, Ekkehard, Dr.**
**Amselweg 14**
**W-6240 Königstein/Taunus (DE)**
Erfinder : **Bickel, Martin, Dr.**
**Mittelstedter Weg 3**
**W-6380 Bad Homburg (DE)**
Erfinder : **Günzler-Pukall, Volkmar, Dr.**
**Gross-Seelheimer Strasse 13**
**W-3550 Marburg (DE)**

EP 0 463 592 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u.a. Fibrosen der Lunge, Leber und Haut (Skleroderma) sowie die Atherosklerose.

Es ist bekannt, daß die Inhibierung der Prolinhydroxylase durch bekannte Inhibitoren wie $\alpha,\alpha'$-Dipyridyl zu einer Hemmung der $C1_q$-Biosynthese von Makrophagen führt (W. Müller et al., FEBS Lett. 90 (1978), 218; Immunbiology 155 (1978), 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß das Enzym Prolinhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625-633).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4-und-2,5-dicarbonsäure, die die Kollagenbiosynthese im Tiermodell wirksam hemmen. So wird in der DE-A 37 03 959 unter anderem die Synthese von N,N'-Bis(2-methoxyethyl)-pyridin-2,4-dicarbonsäurediamid und N,N'-Bis(3-isopropoxypropyl)-pyridin-2,4-dicarbonsäurediamid beschrieben.

In den deutschen Patentanmeldungen P 38 26 471.4 und P 38 28 140.6 wird ein verbessertes Verfahren zur Herstellung von N,N'-Bis(2-methoxyethyl)-pyridin-2,4-dicarbonsäurediamid vorgeschlagen.

Die deutsche Patentanmeldung P 39 24 093.2 schlägt neue N,N'-Bis(alkoxyalkyl)-pyridin-2,4-dicarbonsäurediamide vor.

Die deutsche Patentanmeldung P 40 01 002.3 beschreibt die Verwendung von Pyridin-2,4- und 2,5-dicarbonsäuredi-(nitroxyalkyl)amide zur Herstellung von Prolin- und Lysinhydroxylase hemmenden Arzneimitteln.

Sowohl Pyridin-2,4- und -2,5-dicarbonsäurediamid (Hirakata et al., J. pharm. Soc. Japan 77 (1957) 219 und Häring et al., Helv. 37 (1954) 147, 153) als auch Pyridin-2,4 und -2,5-dicarbonsäuredihydrazid (Itai et al., Bl. nation. hyg. Labor. Tokyo, 74 (1956) 115, 117 und Shinohara et al., Chem. High Polymers Japan, 15 (1958) 839) sind bereits als Tuberkulosemittel bekannt.

In der JP 53/28175 (78/28175) werden N,N'-bis(2-nitrooxyethyl)pyridin-2,4- und -2,5-dicarbonsäurediamide als Substanzen mit vasodilatorischer Wirkung beschrieben. Überraschend wurde nun gefunden, daß 2,4- und 2,5-substituierte Pyridin-N-oxide der unten angegebenen allgemeinen Formel I sowie die physiologisch verträglichen Salze die Lysin- und Prolinhydroxylase im Tiermodell wirksam inhibieren.

Die Erfindung betrifft dementsprechend 2, 4-substituierte Pyridin-N-oxide der allgemeinen Formel I

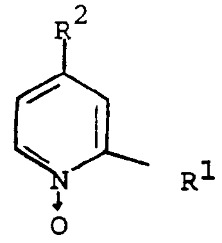

(I)

worin

$R^1$ -C(O)-X-$R^3$ bedeutet, wobei
X -N($R^{3'}$)- bedeutet und

$R^3$ Wasserstoff oder $C_2$ und $C_3$-Alkyl bedeutet, wobei der Alkylrest substituiert ist mit einem oder mehreren gleichen $C_1$- oder $C_2$-Alkoxyresten

und

$R^{3'}$

die Bedeutung von $R^3$ hat,

$R^2$

die Bedeutung von $R^1$ hat, wobei die Reste $R^1$ und $R^2$ gleich oder verschieden sind
sowie die physiologisch verträglichen Salze.

Weiterhin betrifft die Erfindung die Verwendung von Verbindungen der allgemeinen Formel I sowie die physiologisch verträglichen Salze zur Herstellung eines Prolin- und Lysinhydroxylase hemmenden Arzneimittels.

Schließlich betrifft die Erfindung die Verbindungen der allgemeinen Formel I zur Verwendung als Arzneimittel.

Insbesondere betrifft die Erfindung die Verbindungen der Formel I zur Anwendung als Fibrosuppressiva und Immunsuppressiva sowie zur Inhibierung der Prolin- und Lysinhydroxylase und zur Beeinflussung des Stoffwechsels von Kollagen und kollagenähnlichen Stoffen bzw. der Biosynthese von C1q.

Alle genannten Alkylreste mit mehr als 2 C-Atomen können sowohl geradkettig als auch verzweigt sein.

Weiterhin betrifft die Erfindung ein Verfahren zur Herstellung von Verbindungen der allgemeinen Formel I.

Die Herstellung der erfindungsgemäßen Verbindungen gelingt am einfachsten dadurch, daß man Oxidationsmittel wie z.B. Wasserstoffperoxid oder Persäuren wie Peressigsäure, Perfluoressigsäure, Perbenzoesäure oder Metachlorperbenzoesäure in Lösungsmitteln wie chlorierte Kohlenstoffe, wie z.B. Methylenchlorid, Chloroform, Tri- oder Tetrachlorethylen, Benzol oder Toluol zu den zu oxidierenden Pyridinverbindungen, die ebenfalls in den obengenannten Lösungsmitteln gelöst sein können, gibt und bei Temperatur zwischen -30 und +40°C bevorzugt 0 und +25°C zwischen 30 Minuten und 3 Tagen rührt. Die Beendigung der Reaktion läßt sich beispielsweise mittels Dünnschichtchromatographie bestimmen. Vorzugsweise lassen sich die erfindungsgemäßen Verbindungen herstellen, in dem man das Pyridinderivat und das Oxidationsmittel in äquimolaren Mengen oder bis zu einem etwa 5-fachen Überschuß an Oxidationsmittel einsetzt.

Gegebenenfalls kann auch ein Überschuß an Persäure beseitigt werden, in dem man beispielsweise gasförmig Ammoniak in die Reaktionslösung einleitet und den entstehenden Niederschlag durch Filtration von der Reaktionslösung abtrennt.

Gegebenenfalls kann die Aufarbeitung der Produkte beispielsweise durch Extraktion oder durch Chromatographie z.B. über Kieselgel erfolgen. Das isolierte Produkt kann umkristallisiert werden.

Eine allgemeine Vorschrift dieser Oxidationsmethode ist auch beispielsweise in "E. Lingsberg, Pyridine and its Derivatives, Interscience Publishers, New York, 1961, Part 2, 93" beschrieben.

Die Oxidation mit Wasserstoffperoxid ist beispielsweise in "E. Ochiai, J. Org. Chem. 18, 534 (1953)" beschrieben.

Die Darstellung der für die beschriebene Oxidation notwendigen unterschiedlichen Pyridinderivate wird in den schon als Stand der Technik zitierten Patentanmeldungen ausgeführt. Zu nennen sind die deutschen Patentanmeldungen P 38 26 471.4, 38 28 140.6, 39 24 093.2, 40 01 002.3 sowie den DE-A-37 03 959, 37 03 962 und 37 03 963.

Die erfindungsgemäßen Verbindungen der Formel I besitzen wertvolle pharmakologische Eigenschaften und zeigen insbesondere Wirksamkeit als Hemmer der Prolin- und Lysinhydroxylase, als Fibrosuppressivum, Immunsuppressivum und Antiatherosklerotikum.

Die antifibrotische Wirkung kann im Modell der Tetrachlorkohlenstoff-induzierten Leberfibrose bestimmt werden. Dazu werden Ratten mit $CCl_4$ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben - analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentration 1 - 100 mg/kg wirksam.

Die Aktivität der Fibrogenese kann durch radioimmunologische Bestimmung des N-terminalen Propeptids des Kollagens Typ-III oder der N- bzw. C-terminalen Quervernetzungsdomäne des Kollagens-Typ-IV (7s-Kollagen bzw. Typ-IV-Kollagen-$NC_1$) im Serum bestimmt werden.

Zu diesem Zweck wurden die Hydroxyprolin-, Prokollagen-III-Peptid-, 7s-Kollagen- und Typ-IV-Kollagen-$NC_1$-Konzentrationen in der Leber von

a) unbehandelten Ratten (Kontrolle)

b) Ratten, denen Tetrachlorkohlenstoff verabreicht wurde ($CCl_4$-Kontrolle)

c) Ratten, denen zunächst $CCl_4$ und anschließend eine erfindungsgemäße Verbindung verabreicht wurde

gemessen (diese Testmethode wird beschrieben von Rouiller, C., experimental toxic injury of the liver; in The Liver, C. Rouiller, Vol. 2, S. 335-476, New York, Academic Press, 1964).

Ein anderes Modell zur Evaluierung der antibiotischen Wirkung ist das der Bleomycin-induzierten Lungenfibrose wie bei Kelley et al. (J. Lab. Clin. Med. 96, 954, (1980)) beschrieben. Für die Evaluierung der Wirkung der erfindungsgemäßen Verbindungen der Granulationsgewebe kann das Modell des Wattebauschgranuloms, wie bei Meier et al., Experimentia 6, 469 (1950) beschrieben, herangezogen werden. Im folgenden ist die Erfindung anhand von Beispielen näher erläutert.

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 - 25 mg/kg/Tag, vorzugsweise 1 - 5 mg/kg/Tag oder parenteral in Dosen von 0,01 - 5 mg/kg/Tag, vorzugsweise 0,01 - 2,5 mg/kg/Tag, insbesondere 0,5 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimittel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral oder rektal appliziert werden.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige oder ölige Lösungen.

Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung an Hand von Beispielen näher erläutert.

Allgemeine Vorschrift zur Herstellung der Verbindungen

1 Äquivalent Pyridin-Derivat (Herstellung siehe Beschreibung) werden in Methylenchlorid vorgelegt und bei Raumtemperatur 1 Äquivalent Metachlorperbenzoesäure (MCPBA), gelöst in Methylenchlorid, zugetropft. Es wird bei Raumtemperatur gerührt. Nach Beendigung der Reaktion wird solange gasförmiger Ammoniak unter Eiskühlung in die Lösung geblasen, bis kein Niederschlag mehr entsteht. Der Niederschlag wird abfiltriert, das Filtrat mit Magnesiumsulfat getrocknet und eingeengt.

Das Rohprodukt wird umkristallisiert oder mittels Dünnschichtchromatographie gereinigt.

Die in den folgenden Beispielen genannten Verbindungen werden gemäß dieser allgemeinen Vorschrift hergestellt.

Beispiel 1

Pyridin-2,4-dicarbonsäure-di-N,N′-(2-methoxyethyl)amid-N-oxid

Aus 1 g Pyridin-2,4-dicarbonsäure-N,N′-(2-methoxyethyl)amid und 0,62 g MCPBA.

Ausbeute: 620 mg (Chrom.: Ethylacetat/Methanol 5/1) Fp.: 102°C

Beispiel 2

Pyridin-2,4-dicarbonsäure-di-N,N′-(3-methoxypropyl)amid-N-oxid

Aus 1 g Pyridin-2,4-dicarbonsäure-N,N′-(3-methoxypropyl)-amid und 1,2 g MCPBA.

Ausbeute: 0,58 g (Umkristallisation: Ethanol)

Fp.: 90°C

Beispiel 3

Pyridin-2,4-dicarbonsäure-di-N,N′-(2-dimethoxyethyl)amid-N-oxid

Aus 1 g Pyridin-2,4-dicarbonsäure-N,N′-(2-dimethoxyethyl)amid und 1,1 g MCPBA.

Ausbeute: 0,5 g (Chrom.: Ethylacetat/Methanol 5/1)

Fp.: 86°C

Beispiel 4

Pyridin-2,4-dicarbonsäure-di-N,N′-(3-ethoxypropyl)amid-N-oxid

Aus 1 g Pyridin-2,4-dicarbonsäure-di-N,N′-(3-ethoxypropyl)amid und 1,5 g MCPBA.

Ausbeute: 0,34 g (Chrom.: Ethylacetat/Methanol 5/1)

Fp.: 81°C

Beispiel 5

Pharmakologische Wirksamkeit

Um die effiziente Inhibitierung der Prolin-hydroxylase und der Lysin-hydroxylase durch die Verbindungen gemäß der Erfindung zu zeigen, werden die Konzentrationen von Bilirubin, Bile-Säuren und Gamma GT im Serum von

a) unbehandelten Ratten (Kontrolle),

b) mit $CCl_4$ behandelten Ratten,

c) Ratten, denen zuerst $CCl_4$ und anschließend eine

Verbindung gemäß der Erfindung gegeben wurden, gemessen. (Die Methode ist beschrieben von Rouiller, C., Experimental toxic injury of the liver; in The Liver, C. Rouiller, Vol. 2, Seiten 335-476, New York, Academic Press 1964).

Die Ergebnisse sind in Tabelle 1 zusammengefaßt.

Tabelle 1:

Wirkung von Prolyl-hydroxylase-Inhibitoren auf durch $CCl_4$ induzierte Leberfibrose in Ratten

| Behandlung | Dosis[a] mg/kg | N | Bilirubin µm | Bile acids | Gamma GT U/L |
|---|---|---|---|---|---|
| Kontrolle | - | 5 | 1,76 ± 0,27 | 26 ± 6,8 | 2 ± 0 |
| $CCl_4$ | - | 22 | 4,98 ± 1,06 | 81 ± 8,7 | 5,3 ± 1,4 |
| Beispiel 1 | 20 | 12 | 6,30 ± 5,4 (0) | 97 ± 76 (0) | 4,3 ± 3,1 (27) |
| Beispiel 2 | 20 | 11 | 2,90 ± 0,94* (65) | 71 ± 42 (18) | 3,3 ± 2,2* (59) |

Die Resultate sind Mittelwerte ± Standardabweichung,
*p <0,05 gegen $CCl_4$-Behandlung,
Werte in Klammern bedeuten die prozentuale Verbesserung gegenüber einer ausschließlichen $CCl_4$-Behandlung.
a: totale tägliche orale Dosis.

**Patentansprüche**

**Patentansprüche für folgende Vertragsstaaten : DE, GB, FR, IT, NL, SE, LI, CH, BE, AT, LU, GR, DK**

1. 2,4-substituierte Pyridin-N-oxide der Formeln I

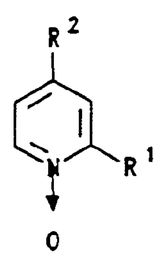

(I)

worin

$R^1$ -C(O)-X-$R^3$ bedeutet, wobei X -N($R^{3'}$)- bedeutet und

$R^3$ Wasserstoff oder $C_1$-$C_5$-Alkyl bedeutet, wobei der Alkylrest substituiert ist mit einem oder zwei gleichen $C_1$- oder $C_2$-Alkoxyresten

und

$R^{3'}$ die Bedeutung von $R^3$ hat,

$R^2$ die Bedeutung von $R^1$ hat, wobei die Reste $R^1$ und $R^2$ gleich oder verschieden sind sowie die physiologisch verträglichen Salze.

**2.** Verfahren zur Herstellung von Verbindungen der Formel I gemäß Anspruch 1, dadurch gekennzeichnet, daß man

a) eine Verbindung der Formel III

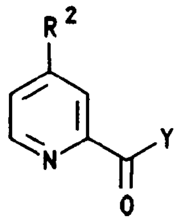

(III)

worin Y Halogen, Hydroxy oder Alkoxy bedeutet mit einer Verbindung der Formel IV

H-X-$R^3$ (IV)

worin X und $R^3$ die in Anspruch 1 angegebenen Bedeutungen haben, umsetzt oder daß man

b) eine Verbindung der Formel V

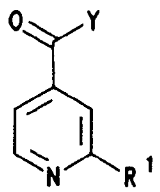

(V)

worin Y Halogen, Hydroxy oder Alkoxy bedeutet, mit einer Verbindung der Formel VI

H-X-$R^3$ (VI)

worin X und $R^3$ die in Anspruch 1 angegebenen Bedeutungen haben, umsetzt,

daß man gegebenenfalls in der Seitenkette $R^3$ einen weiteren Substituenten einführt und daß man anschließend die so erhaltene Verbindung in das N-Oxid überführt und gegebenenfalls anschließend die so erhaltene Verbindung in ein physiologisch verträgliches Salz überführt.

**3.** 2,4-substituierte Pyridin-N-Oxide der Formel I

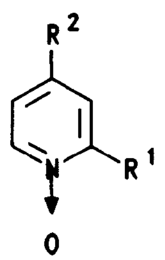

(I)

worin

$R^1$ -C(O)-X-$R^3$ bedeutet, wobei

X -N($R^{3'}$)- bedeutet und

$R^3$ Wasserstoff oder $C_2$- und $C_3$-Alkyl bedeutet, wobei der Alkylrest substituiert ist mit einem oder zwei gleichen $C_1$- oder $C_2$-Alkoxyresten

und

$R^{3'}$ die Bedeutung von $R^3$ hat,

$R^2$ die Bedeutung von $R^1$ hat, wobei die Reste $R^1$ und $R^2$ gleich oder verschieden sind

sowie die physiologisch verträglichen Salze, zur Anwendung als Arzneimittel.

**4.** 2,4-substituierte Pyridin-N-oxide der Formeln I und I′

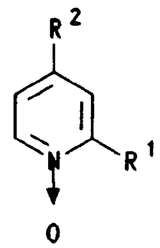

(I)

worin

$R^1$ -C(O)-X-$R^3$ bedeutet, wobei

X -N($R^{3'}$)- bedeutet und

$R^3$ Wasserstoff oder $C_2$- und $C_3$-Alkyl bedeutet, wobei der Alkylrest substituiert ist mit einem oder zwei gleichen $C_1$-$C_2$-Alkoxyresten

und

$R^{3'}$ die Bedeutung von $R^3$ hat,

$R^2$ die Bedeutung von $R^1$ hat, wobei die Reste $R^1$ und $R^2$ gleich oder verschieden sind

sowie die physiologisch verträglichen Salze, zur Inhibierung der Prolin- und Lysinhydroxylase.

5. 2,4-substituierte Pyridin-N-oxide der Formeln I und I′

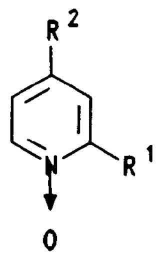

(I)

worin

$R^1$ -C(O)-X-$R^3$ bedeutet, wobei

X -N($R^{3'}$)- bedeutet und

$R^3$ Wasserstoff oder $C_2$- und $C_3$-Alkyl bedeutet, wobei substituiert ist mit einem oder zwei gleichen $C_1$- oder $C_2$-Alkoxyresten

und

$R^{3'}$ die Bedeutung von $R^3$ hat,

$R^2$ die Bedeutung von $R^1$ hat, wobei die Reste $R^1$ und $R^2$ gleich oder verschieden sind

sowie die physiologisch verträglichen Salze, zur Anwendung als Fibrosuppressiva und Immunisuppressiva.

6. Pharmazeutische Zusammensetzung, enthaltend eine Verbindung der Formel I gemäß Anspruch 3 und einen pharmazeutisch verträglichen Träger.

7. Verwendung von Verbindungen der Formel I gemäß Anspruch 3 zur Beeinflussung des Stoffwechsels von Collagen und collagenähnlichen Stoffen bzw. der Biosynthese von $Cl_q$.

8. Verwendung von Verbindungen der Formel I gemäß Anspruch 3 zur Behandlung von Störungen des Stoffwechsels von Collagen und collagenähnlichen Stoffen bzw. der Biosynthese von $Cl_q$.

9. Verfahren zur Herstellung einer pharmazeutischen Zusammensetzung gemäß Anspruch 6, dadurch gekennzeichnet, daß man eine Verbindung der Formel I gemäß Anspruch 1 und einen pharmazeutisch verträglichen Träger in eine geeignete Darreichungsform überführt.

**Patentansprüche für folgenden Vertragsstaat : ES**

1. Verfahren zur Herstellung von 2,4-substituierten Pyridin-N-oxiden der Formel I

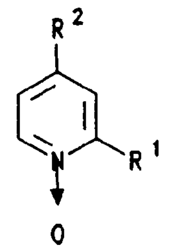

(I)

worin
$R^1$ -C(O)-X-$R^3$ bedeutet, wobei
X -N($R^{3'}$)- bedeutet und
$R^3$ Wasserstoff oder $C_1$-$C_5$-Alkyl bedeutet, wobei der Alkylrest substituiert ist mit einem oder zwei gleichen $C_1$- oder $C_2$-Alkoxyresten
und
$R^{3'}$ die Bedeutung von $R^3$ hat,
$R^2$ die Bedeutung von $R^1$ hat, wobei die Reste $R^1$ und $R^2$ gleich oder verschieden sind
sowie die physiologisch verträglichen Salze.

2. Verfahren zur Herstellung von Verbindungen der Formel I gemäß Anspruch 1, dadurch gekennzeichnet, daß man
a) eine Verbindung der Formel III

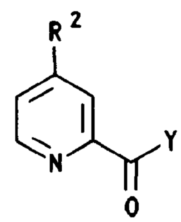

(III)

worin Y Halogen, Hydroxy oder Alkoxy bedeutet mit einer Verbindung der Formel IV

H-X-$R^3$ (IV)

worin X und $R^3$ die in Anspruch 1 angegebenen Bedeutungen haben, umsetzt oder daß man
b) eine Verbindung der Formel V

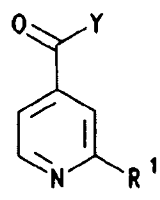

(V)

worin Y Halogen, Hydroxy oder Alkoxy bedeutet, mit einer Verbindung der Formel VI

H-X-$R^3$ (VI)

worin X und $R^3$ die in Anspruch 1 angegebenen Bedeutungen haben, umsetzt,
daß man gegebenenfalls in der Seitenkette $R^3$ einen weiteren Substituenten einführt und daß man anschließend die so erhaltene Verbindung in das N-Oxid überführt und gegebenenfalls anschließend die so erhaltene Verbindung in ein physiologisch verträgliches Salz überführt.

3. 2,4-substituierte Pyridin-N-Oxide der Formel I

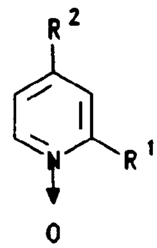

( I )

worin
$R^1$ -C(O)-X-$R^3$ bedeutet, wobei
X -N($R^{3'}$)- bedeutet und
$R^3$ Wasserstoff oder $C_2$- und $C_3$-Alkyl bedeutet, wobei der Alkylrest substituiert ist mit einem oder zwei gleichen $C_1$- oder $C_2$-Alkoxyresten
und
$R^{3'}$ die Bedeutung von $R^3$ hat,
$R^2$ die Bedeutung von $R^1$ hat, wobei die Reste $R^1$ und $R^2$ gleich oder verschieden sind
sowie die physiologisch verträglichen Salze, zur Anwendung als Arzneimittel.

4. Verfahren zur Herstellung von 2,4-substituierten Pyridin-N-oxiden der Formel I

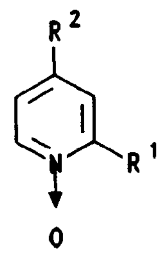

( I )

worin
$R^1$ -C(O)-X-$R^3$ bedeutet, wobei
X -N($R^{3'}$)- bedeutet und
$R^3$ Wasserstoff oder $C_2$- und $C_3$-Alkyl bedeutet wobei, der Alkylrest substituiert ist mit einem oder zwei gleichen $C_1$ oder $C_2$-Alkoxyresten
und
$R^{3'}$ die Bedeutung von $R^3$ hat,
$R^2$ die Bedeutung von $R^1$ hat, wobei die Reste $R^1$ und $R^2$ gleich oder verschieden sind
sowie die physiologisch verträglichen Salze, zur Inhibierung der Prolin- und Lysinhydroxylase.

5. Verfahren zur Herstellung von 2,4-substituierte Pyridin-N-oxide der Formel I

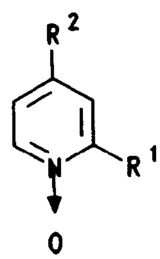

( I )

worin
$R^1$ -C(O)-X-$R^3$ bedeutet, wobei
X -N($R^{3'}$)- bedeutet und
$R^3$ Wasserstoff oder $C_2$- und $C_3$-Alkyl bedeutet, wobei der Alkylrest substituiert ist mit einem oder

zwei gleichen $C_1$- oder $C_2$-Alkoxyresten
und
$R^{3'}$ die Bedeutung von $R^3$ hat,
$R^2$ die Bedeutung von $R^1$ hat, wobei die Reste $R^1$ und $R^2$ gleich oder verschieden sind
sowie die physiologisch verträglichen Salze, zur Anwendung als Fibrosuppressiva und Immun suppressiva.

6. Pharmazeutische Zusammensetzung, enthaltend eine Verbindung der Formel I gemäß Anspruch 3 und einen pharmazeutisch verträglichen Träger.

7. Verwendung von Verbindungen der Formel I gemäß Anspruch 3 zur Beeinflussung des Stoffwechsels von Collagen und collagenähnlichen Stoffen bzw. der Biosynthese von $Cl_q$.

8. Verwendung von Verbindungen der Formel I gemäß Anspruch 3 zur Behandlung von Störungen des Stoffwechsels von Collagen und collagenähnlichen Stoffen bzw. der Biosynthese von $Cl_q$.

9. Verfahren zur Herstellung einer pharmazeutischen Zusammensetzung gemäß Anspruch 6, dadurch gekennzeichnet, daß man eine Verbindung der Formel I gemäß Anspruch 1 und einen pharmazeutisch verträglichen Träger in eine geeignete Darreichungsform überführt.

## Claims

### Claims for the following Contracting State : ES

1. A process for the preparation of 2,4-substituted pyridine-N-oxides of the formula I

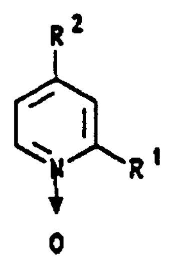

(I)

in which
$R^1$ is -C(O)-X-$R^3$, where
X is -N($R^{3'}$)- and
$R^3$ is hydrogen or $C_1$- to $C_5$-alkyl, where the alkyl radical is substituted by one or two identical $C_1$- or $C_2$-alkoxy radicals
and
$R^{3'}$ has the meaning of $R^3$,
$R^2$ has the meaning of $R^1$, where the radicals $R^1$ and $R^2$ are identical or different
or the physiologically tolerable salts.

2. A process for the preparation of compounds of the formula I as claimed in claim 1, which comprises
a) reacting a compound of the formula III

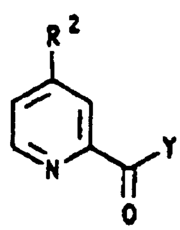

(III)

in which Y is halogen, hydroxyl or alkoxy, with a compound of the formula IV

H-X-$R^3$ (IV)

in which X and $R^3$ have the meanings indicated in claim 1, or

b) reacting a compound of the formula V

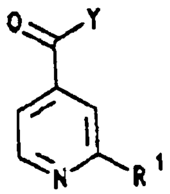

(V)

in which Y is halogen, hydroxyl or alkoxy, with a compound of the formula VI

H-X-$R^3$ (VI)

in which X and $R^3$ have the meanings indicated in claim 1,
optionally introducing a further substituent into the side chain $R^3$ and then converting the compound thus obtained to the N-oxide and optionally then converting the compound thus obtained to a physiologically tolerable salt.

**3.** A process for the preparation of 2,4-substituted pyridine-N-oxide of the formula I

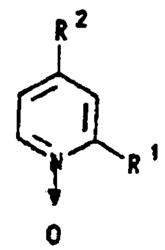

(I)

in which

$R^1$ is -C(O)-X-$R^3$, where

X is -N($R^{3'}$)- and

$R^3$ is hydrogen or $C_2$- or $C_3$-alkyl, where the alkyl radical is substituted by one or two identical $C_1$- or $C_2$-alkoxy radicals

and

$R^{3'}$ has the meaning of $R^3$,

$R^2$ has the meaning of $R^1$, where the radicals $R^1$ and $R^2$ are identical or different

or the physiologically tolerable salts, for use as a pharmaceutical.

**4.** A process for the preparation of 2,4-substituted pyridine-N-oxide of the formula I in which

$R^1$ is -C(O)-X-$R^3$, where

X is -N($R^{3'}$)- and

$R^3$ is hydrogen or $C_2$- or $C_3$-alkyl, where the alkyl radical is substituted by one or two identical $C_1$ or $C_2$-alkoxy radicals

and

$R^{3'}$ has the meaning of $R^3$,

$R^2$ has the meaning of $R^1$, where the radicals $R^1$ and $R^2$ are identical or different

or the physiologically tolerable salts, for inhibiting proline hydroxylase and lysine hydroxylase.

**5.** A process for the preparation of 2,4-substituted pyridine-N-oxide of the formula I

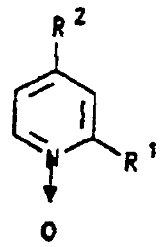

(I)

in which

$R^1$ is -C(O)-X-$R^3$, where

X is -N($R^{3'}$)- and

$R^3$ is hydrogen or $C_2$- or $C_3$-alkyl, where the alkyl radical is substituted by one or two identical $C_1$ or $C_2$-alkoxy radicals

and

$R^{3'}$ has the meaning of $R^3$,

$R^2$ has the meaning of $R^1$, where the radicals $R^1$ and $R^2$ are identical or different

or the physiologically tolerable salts, for use as fibrosuppressants and immunosuppressants.

6. A pharmaceutical composition, containing a compound of the formula I as in claim 3, and a pharmaceutically tolerable excipient.

7. The use of compounds of the formula I as in claim 3 for affecting the metabolism of collagen and collagen-like substances or the biosynthesis of $C1_q$.

8. The use of compounds of the formula I as in claim 3 for the treatment of disorders of the metabolism of collagen and collagen-like substances or the biosynthesis of $C1_q$.

9. A process for the production of a pharmaceutical composition as claimed in claim 6, which comprises converting a compound of the formula I as claimed in claim 1 and a pharmaceutically tolerable excipient into a suitable administration form.

**Claims for the following Contracting States : DE, GB, FR, IT, NL, SE, LI, CH, BE, AT, LU, GR, DK**

1. A 2,4-substituted pyridine-N-oxide of the formula I

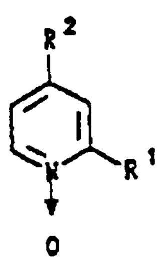

(I)

in which

$R^1$ is -C(O)-X-$R^3$, where

X is -N($R^{3'}$)- and

$R^3$ is hydrogen or $C_1$- to $C_5$-alkyl, where the alkyl radical is substituted by one or two identical $C_1$- or $C_2$-alkoxy radicals

and

$R^{3'}$ has the meaning of $R^3$,

$R^2$ has the meaning of $R^1$, where the radicals $R^1$ and $R^2$ are identical or different

or the physiologically tolerable salts.

2. A process for the preparation of compounds of the formula I as claimed in claim 1, which comprises
a) reacting a compound of the formula III

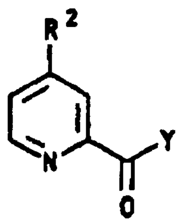

(III)

in which Y is halogen, hydroxyl or alkoxy, with a compound of the formula IV

$$H\text{-}X\text{-}R^3 \qquad (IV)$$

in which X and $R^3$ have the meanings indicated in claim 1, or
b) reacting a compound of the formula V

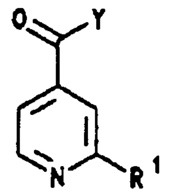

(V)

in which Y is halogen, hydroxyl or alkoxy, with a compound of the formula VI

$$H\text{-}X\text{-}R^3 \qquad (VI)$$

in which X and $R^3$ have the meanings indicated in claim 1,
optionally introducing a further substituent into the side chain $R^3$ and then converting the compound thus obtained to the N-oxide and optionally then converting the compound thus obtained to a physiologically tolerable salt.

3. A 2,4-substituted pyridine-N-oxide of the formula I

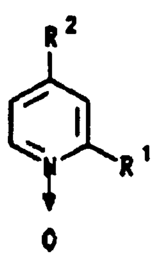

(I)

in which
$R^1$ is -C(O)-X-$R^3$, where
X is -N($R^{3'}$)- and
$R^3$ is hydrogen or $C_2$- or $C_3$-alkyl, where the alkyl radical is substituted by one or two identical $C_1$- or $C_2$-alkoxy radicals
and
$R^{3'}$ has the meaning of $R^3$,
$R^2$ has the meaning of $R^1$, where the radicals $R^1$ and $R^2$ are identical or different
or the physiologically tolerable salts, for use as a pharmaceutical.

4. A 2,4-substituted pyridine-N-oxide of the formula I

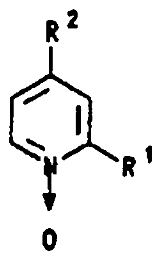

(I)

in which

$R^1$ is -C(O)-X-$R^3$, where

X is -N($R^{3'}$)- and

$R^3$ is hydrogen or $C_2$- or $C_3$-alkyl, where the alkyl radical is substituted by one or two identical $C_1$-$C_2$-alkoxy radicals

and

$R^{3'}$ has the meaning of $R^3$,

$R^2$ has the meaning of $R^1$, where the radicals $R^1$ and $R^2$ are identical or different

or the physiologically tolerable salts, for inhibiting proline hydroxylase and lysine hydroxylase.

**5.** A 2,4-substituted pyridine-N-oxide of the formula I

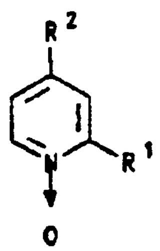

(I)

in which

$R^1$ is -C(O)-X-$R^3$, where

X is -N($R^{3'}$)- and

$R^3$ is hydrogen or $C_2$- or $C_3$-alkyl, where the alkyl radical is substituted by one or two identical $C_1$ or $C_2$-alkoxy radicals

and

$R^{3'}$ has the meaning of $R^3$,

$R^2$ has the meaning of $R^1$, where the radicals $R^1$ and $R^2$ are identical or different

or the physiologically tolerable salts, for use as fibrosuppressants and immunosuppressants.

**6.** A pharmaceutical composition, containing a compound of the formula I as claimed in claim 3, and a pharmaceutically tolerable excipient.

**7.** The use of compounds of the formula I as claimed in claim 3 for affecting the metabolism of collagen and collagen-like substances or the biosynthesis of $C1_q$.

**8.** The use of compounds of the formula I as claimed in claim 3 for the treatment of disorders of the metabolism of collagen and collagen-like substances or the biosynthesis of $C1_q$.

**9.** A process for the production of a pharmaceutical composition as claimed in claim 6, which comprises converting a compound of the formula I as claimed in claim 1 and a pharmaceutically tolerable excipient into a suitable administration form.

## Revendications

**Revendications pour les Etats contractants suivants : DE, GB, FR, IT, NL, SE, LI, CH, BE, AT, LU, GR, DK**

1. N-oxydes de pyridine 2,4-substitués de formule I

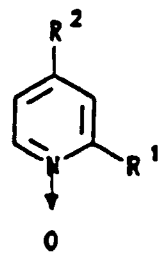

(I)

dans laquelle
$R^1$ représente -C(O)-X-$R^3$,
X représentant -N($R^{3'}$) et
$R^3$ représentant un atome d'hydrogène ou un radical alkyle en $C_1$-$C_5$, le radical alkyle étant substitué par un ou deux groupes alcoxy en $C_1$ ou $C_2$ identiques,
et
$R^{3'}$ ayant la signification de $R^3$,
$R^2$ a la signification de $R^1$, les radicaux $R^1$ et $R^2$ étant identiques ou différents.
et sels physiologiquement acceptables.

2. Procédé pour la préparation des composés de formule I selon la revendication 1, caractérisé en ce que
a) on fait réagir un composé de formule III

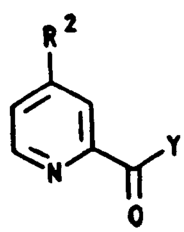

(III)

dans laquelle Y est un atome d'halogène ou un groupe hydroxy ou alcoxy, avec un composé de formule IV

H-X-$R^3$ (IV)

dans laquelle X et $R^3$ ont les significations données dans la revendication 1, ou en ce que
b) on fait réagir un composé de formule V

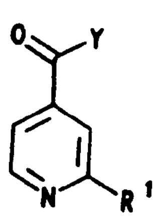

(V)

dans laquelle Y représente un atome d'halogène ou un groupe hydroxy ou alcoxy, avec un composé de formule VI

H-X-$R^3$ (VI)

dans laquelle X et $R^3$ ont les significations données dans la revendication 1,
en ce que éventuellement on introduit un autre substituant dans la chaîne latérale $R^3$ et en ce que l'on

convertit le composé ainsi obtenu en le N-oxyde et éventuellement on convertit ensuite le composé ainsi obtenu en un sel physiologiquement acceptable.

**3.** N-oxydes de pyridine 2,4-substitués de formule I

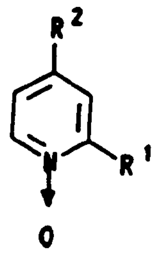

(I)

dans laquelle

$R^1$ représente -C(O)-X-$R^3$,

X représentant -N($R^{3'}$) et

$R^3$ représentant un atome d'hydrogène ou un radical alkyle en $C_2$-$C_3$, le radical alkyle étant substitué par un ou deux groupes alcoxy en $C_1$ ou $C_2$ identiques,

et

$R^{3'}$ ayant la signification de $R^3$,

$R^2$ a la signification de $R^1$, les radicaux $R^1$ et $R^2$ étant identiques ou différents.

et sels physiologiquement acceptables, pour utilisation en tant que médicament.

**4.** N-oxydes de pyridine 2,4-substitués de formules I et I'

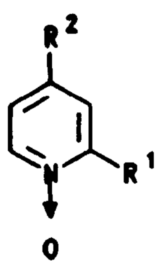

(I)

dans laquelle

$R^1$ représente -C(O)-X-$R^3$,

X représentant -N($R^{3'}$) et

$R^3$ représentant un atome d'hydrogène ou un radical alkyle en $C_2$-$C_3$, le radical alkyle étant substitué par un ou deux groupes alcoxy en $C_1$ ou $C_2$ identiques,

et

$R^{3'}$ ayant la signification de $R^3$,

$R^2$ a la signification de $R^1$, les radicaux $R^1$ et $R^2$ étant identiques ou différents.

et sels physiologiquement acceptables, pour l'inhibition de la proline hydroxylase et de la lysine hydroxylase.

**5.** N-oxydes de pyridine 2,4-substitués de formule I et I'

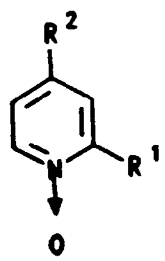

(I)

dans laquelle

$R^1$ représente -C(O)-X-$R^3$,

X représentant -N($R^{3'}$) et

$R^3$ représentant un atome d'hydrogène ou un radical alkyle en $C_2$-$C_3$, le radical alkyle étant substitué par un ou deux groupes alcoxy en $C_1$ ou $C_2$ identiques, et

$R^{3'}$ ayant la signification de $R^3$,

$R^2$ a la signification de $R^1$, les radicaux $R^1$ et $R^2$ étant identiques ou différents.

et sels physiologiquement acceptables, pour utilisation en tant que fibrosuppresseurs et immunosuppresseurs.

6. Composition pharmaceutique contenant un composé de formule I selon la revendication 3 et un véhicule pharmaceutiquement acceptable.

7. Utilisation de composés de formule I selon la revendication 3, pour influencer le métabolisme du collagène et de substances analogues au collagène ou la biosynthèse du C1q.

8. Utilisation de composés de formule I selon la revendication 3, pour le traitement de troubles du métabolisme du collagène et de substances analogues au collagène ou de la biosynthèse du C1q.

9. Procédé pour la préparation d'une composition pharmaceutique selon la revendication 6, caractérisé en ce que l'on convertit en une forme d'administration appropriée un composé de formule 1 selon la revendication 1 et un véhicule pharmaceutiquement acceptable.

**Revendications pour l'Etat contractant suivant : ES**

1. Procédé pour la préparation de N-oxydes de pyridine 2,4-substitués de formule I

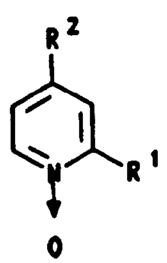

(I)

dans laquelle

$R^1$ représente -C(O)-X-$R^3$,

X représentant -N($R^{3'}$) et

$R^3$ représentant un atome d'hydrogène ou un radical alkyle en $C_1$-$C_5$, le radical alkyle étant substitué par un ou deux groupes alcoxy en $C_1$ ou $C_2$ identiques,

et

$R^{3'}$ ayant la signification de $R^3$,

$R^2$ a la signification de $R^1$, les radicaux $R^1$ et $R^2$ étant identiques ou différents.

ainsi que des sels physiologiquement acceptables.

2. Procédé pour la préparation des composés de formule I selon la revendication 1, caractérisé en ce que

a) on fait réagir un composé de formule III

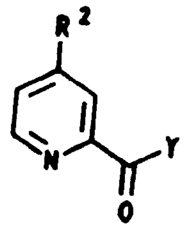

(III)

dans laquelle Y est un atome d'halogène ou un groupe hydroxy ou alcoxy, avec un composé de formule IV

$$H\text{-}X\text{-}R^3 \qquad (IV)$$

dans laquelle X et $R^3$ ont les significations données dans la revendication 1, ou en ce que

b) on fait réagir un composé de formule V

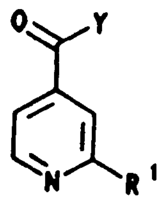

(V)

dans laquelle Y représente un atome d'halogène ou un groupe hydroxy ou alcoxy, avec un composé de formule VI

$$H\text{-}X\text{-}R^3 \qquad (VI)$$

dans laquelle X et $R^3$ ont les significations données dans la revendication 1,

en ce que éventuellement on introduit un autre substituant dans la chaîne latérale $R^3$ et en ce que l'on convertit le composé ainsi obtenu en le N-oxyde et éventuellement on convertit ensuite le composé ainsi obtenu en un sel physiologiquement acceptable.

**3.** N-oxydes de pyridine 2,4-substitués de formule I

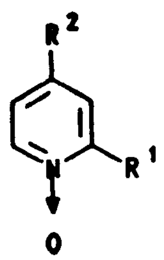

(I)

dans laquelle

$R^1$ représente -C(O)-X-$R^3$,

X représentant -N($R^{3'}$) et

$R^3$ représentant un atome d'hydrogène ou un radical alkyle en $C_2$-$C_3$, le radical alkyle étant substitué par un ou deux groupes alcoxy en $C_1$ ou $C_2$ identiques,

et

$R^{3'}$ ayant la signification de $R^3$,

$R^2$ a la signification de $R^1$, les radicaux $R^1$ et $R^2$ étant identiques ou différents.

et sels physiologiquement acceptables, pour utilisation en tant que médicament.

**4.** Procédé pour la préparation de N-oxydes de pyridine 2,4-substitués de formule I

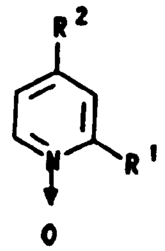

(I)

dans laquelle

$R^1$ représente -C(O)-X-$R^3$,

X représentant -N($R^{3'}$) et

$R^3$ représentant un atome d'hydrogène ou un radical alkyle en $C_2$-$C_3$, le radical alkyle étant substitué par un ou deux groupes alcoxy en $C_1$ ou $C_2$ identiques,

et

$R^{3'}$ ayant la signification de $R^3$,

$R^2$ a la signification de $R^1$, les radicaux $R^1$ et $R^2$ étant identiques ou différents.

ainsi que des physiologiquement acceptables, pour l'inhibition de la proline hydroxylase et de la lysine hydroxylase.

5. Procédé pour la préparation de N-oxydes de pyridine 2,4-substitués de formule I

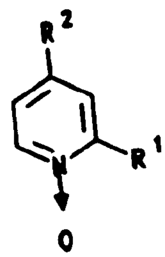

(I)

dans laquelle

$R^1$ représente -C(O)-X-$R^3$,

X représentant -N($R^{3'}$) et

$R^3$ représentant un atome d'hydrogène ou un radical alkyle en $C_2$-$C_3$, le radical alkyle étant substitué par un ou deux groupes alcoxy en $C_1$ ou $C_2$ identiques, et

$R^{3'}$ ayant la signification de $R^3$,

$R^2$ a la signification de $R^1$, les radicaux $R^1$ et $R^2$ étant identiques ou différents.

ainsi que des sels physiologiquement acceptables, pour utilisation en tant que fibrosuppresseurs et immunosuppresseurs.

6. Composition pharmaceutique contenant un composé de formule I selon la revendication 3 et un véhicule pharmaceutiquement acceptable.

7. Utilisation de composés de formule I selon la revendication 3, pour influencer le métabolisme du collagène et de substances analogues au collagène ou la biosynthèse du C1q.

8. Utilisation de composés de formule I selon la revendication 3, pour le traitement de troubles du métabolisme du collagène et de substances analogues au collagène ou de la biosynthèse du C1q.

9. Procédé pour la préparation d'une composition pharmaceutique selon la revendication 6, caractérisé en ce que l'on convertit en une forme d'administration appropriée un composé de formule 1 selon la revendication 1 et un véhicule pharmaceutiquement acceptable.